# FASCICULE DE BREVET EUROPEEN

(11) **EP 0 579 523 B1**
(45) Date de publication et mention de la délivrance du brevet: **05.09.2001**
(21) Numéro de dépôt: 93401665.0
(22) Date de dépôt: 28.06.1993
(51) Int. Cl.: A61F 2/06

(54) **Dispositif formant prothèse vasculaire utilisable pour le traitement des anévrismes**
Gefässprothese zur Behandlung eines aneurysmas
Vascular prosthesis for aneurisma treatment

(30) Priorité: 09.07.1992 FR 9208533
(43) Date de publication de la demande: 19.01.1994
(73) Titulaire: B. Braun Medical SAS, 92100 Boulogne Billancourt (FR); Anidjar, Samy, Dr., 75003 Paris (FR)
(72) Inventeur: Cottenceau, Jean-Philippe, F-92160 Antony (FR); Chevillon, Gérard, F-92130 Montrouge (FR); Anidjar, Samy, F-75003 Paris (FR)
(74) Mandataire: Lerner, François

(56) Documents cités:
- EP-A- 0 421 729
- EP-A- 0 472 731
- WO-A-92/06734
- FR-A- 2 671 280
- US-A- 4 562 596

## Description

L'invention se rapporte à un dispositif réalisé en matière biocompatible pour pouvoir être introduit dans un corps vivant

Plus spécifiquement, est concernée une prothèse avantageusement autoexpansible, implantable dans une zone d'un vaisseau dudit corps pour y renforcer ce vaisseau, ceci dans le cadre du traitement des anévrismes.

FR-A-2 512 678 et US-A-4 562 596 font référence au traitement d'anévrismes.

Pour l'implantation d'un dispositif conforme à l'invention, deux techniques de pose sont actuellement courantes : soit une pose par voie percutanée en utilisant la méthode développée par SELDINGER, soit une pose après avoir dénudé et ouvert chirurgicalement une voie d'accès jusqu'au vaisseau.

A partir de WO-A-92/06734, il est connu un dispositif implantable en percutanée pour le traitement d'un anévrisme, présentant un axe et comprenant
- un manchon souple, allongé présentant un passage intérieur pour canaliser à travers lui le sang,
- des moyens-ressorts liés à des moyens-supports du manchon, pour déployer radialement le manchon et ces moyens-supports, depuis un premier état radialement resserré vers un second état radialement écarté, les moyens-ressorts étant disposés à au moins deux étages axialement éloignés entre lesquels s'étendent les moyens-supports en plusieurs endroits régulièrement répartis autour de l'axe, certains au moins desdits moyens-ressorts présentant une configuration fermée en zigzag définissant un cylindre annulaire axial.

Parmi les "stents" vasculaires, comme dans EP-A-421 729, est également connu un dispositif ou implant comprenant des moyens-ressorts disposés en au moins deux endroits axialement éloignés l'un par rapport à l'autre et liés entre eux par des pattes, en plusieurs endroits répartis autour de l'axe, les moyens-ressorts étant déployables radialement, pour le déploiement radial desdites pattes et de l'ensemble du dispositif, depuis un premier état radialement resserré vers un second état radialement écarté, certains au moins desdits moyens-ressorts présentant une configuration fermée en zigzags définissant un cylindre annulaire axial.

Un problème qui se pose concerne la possibilité d'adapter l'implant à la courbure du vaisseau.

Dans WO-A-92/06734, on disjoint pour cela deux niveaux adjacents d'éléments-ressorts en zigzags que l'on réunit uniquement en certains endroits par des barrettes de liaison, et, à l'endroit de ces "discontinuités" entre deux niveaux, on utilise le manchon extérieur comme articulation locale.

Dans EP-A-421 729, on place un élément flexible d'articulation (tel qu'un ressort) entre deux niveaux adjacents d'éléments-ressorts radiaux en zigzags, également disjoints, sauf à l'endroit d'une liaison filamentaire unique sur laquelle on prévoit cet "élément flexible" d'articulation.

Face à cet art antérieur, on peut noter qu'il demeure un problème non résolu d'une articulation plus précise que celle recourant à un manchon comme dans WO-A-92/06734 ou à une sorte de "ligne d'articulation" unique ou de "colonne vertébrale" comme dans EP-A-421 729.

Aussi, la solution de l'invention pour améliorer la flexibilité de l'implant en particulier dans les vaisseaux tortueux, consiste-t-elle en ce que plusieurs parmi les moyens-supports présentent longitudinalement plusieurs tronçons, deux tronçons successifs adjacents étant réunis entre eux par des moyens d'articulation, ou par des zones intermédiaires d'articulation, indépendant(e)s du manchon souple.

Parmi les avantages, on peut noter:
- une précision accrue lors de la flexion, de par la présence de plusieurs liaisons périphériques entre deux niveaux de moyens-ressorts, avec "intégration" des moyens (ou zones) d'articulation ;
- un maintien plus efficace du manchon auquel les moyens-supports participent, entre deux niveaux successifs de moyens-ressorts ;
- une liberté quant à la position angulaire de l'implant dans son dispositif introducteur. La rotation du cathéter introducteur n'entraîne en outre aucun effet vis-à-vis d'une position angulaire privilégiée du stent dans le vaisseau (à la différence de EP-A-421 729).

Une description plus détaillée de l'invention suit, en référence aux dessins annexés dans lesquels :
- la figure 1 est une vue générale schématique en perspective d'un mode possible de réalisation du dispositif de l'invention,
- la figure 2 est une vue de détail d'une possible conformation de la zone repérée II du dispositif de la figure 1,
- la figure 3 montre en vue partielle, à plus petite échelle, une variante de réalisation des pattes,
- la figure 4 montre à la même échelle une autre réalisation de ces mêmes pattes,
- les figures 5 et 6 montrent des moyens annulaires d'expansion radiale en forme de zigzags, dans deux états,
- la figure 7 montre une prothèse en position déployée, implantée dans un vaisseau, et
- la figure 8 est une vue agrandie du détail VIII de la figure 7.

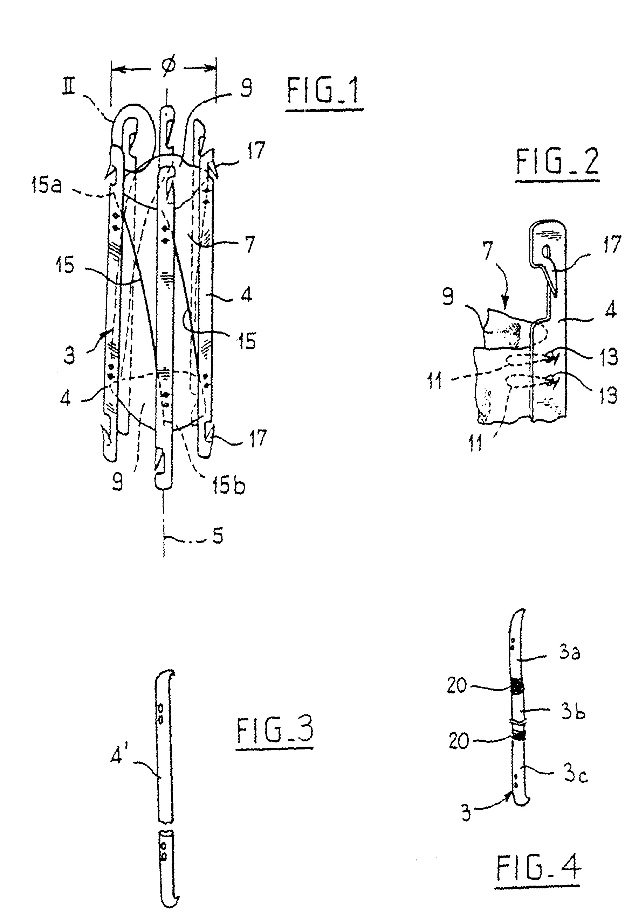

En se reportant d'abord à la figure 1, le dispositif implantable, en particulier par voie percutanée, est en l'espèce destiné à servir de prothèse vasculaire pour remplacer localement, sur une longueur donnée, une paroi de vaisseau dans le cadre en particulier du traitement d'un anévrisme.

Compte tenu de son utilisation dans un corps vivant, et tout particulièrement un corps humain, la prothèse de l'invention sera dans son ensemble réalisée en des matériaux biocompatibles et sera dimensionnée pour pouvoir être implantée jusqu'à la zone du vaisseau choisi.

Dans la version représentée, les pattes de stabilisation 3 consistent en une série de lames ou barrettes 4 parallèles entre elles, de manière à pouvoir être disposées sensiblement parallèlement à l'axe fictif 5 d'un vaisseau.

Les lames 4 seront de préférence relativement peu épaisses (quelques dixièmes de millimètre) mais relativement larges (de l'ordre du millimètre, voire de quelques millimètres) de manière à pouvoir être flexibles tout en assurant un maintien latéral efficace d'un manchon 7 fixé à elles et dont le rôle va donc être de guider le flux sanguin sur la longueur du vaisseau "à réparer".

Une forme des pattes en fil rond métallique droit formant tige 4' de quelques dixièmes à un millimètre de diamètre serait toutefois également adaptée (voir figure 3).

Le manchon 7 sera, de manière classique, pourvu d'un passage intérieur 9 et sera constitué de préférence avec une membrane en tissu tel que du polyéthylènetéréphtalate (PET), voire en tout autre tissu élastiquement déformable.

De préférence, le manchon 7 sera entouré par les lames 4 régulièrement réparties autour de lui, sur toute sa longueur, et auxquelles il pourra être fixé, au moins vers ses extrémités et celles desdites lames, par des fils de suture 11 pouvant traverser des orifices 13 (voir figure 2).

Pour autoriser le déploiement ou le repliement de la prothèse nécessaire à sa mise en place dans le vaisseau, celle-ci a été équipée de moyens à ressort, tels que 15, pour faire passer les pattes-supports 3 d'un premier état radialement rapproché, jusqu'à un second état où ces pattes sont plus écartées les unes des autres avec un diamètre ⌀ supérieur (figure 1), déployant ainsi le manchon 7 pour ouvrir son passage interne 9, le repliement inverse de la prothèse s'effectuant par sollicitation inverse des moyens-ressorts, dans le sens du rapprochement des pattes.

Pour la fixation de la prothèse, une fois celle-ci introduite jusqu'à sa zone d'implantation et déployée, des moyens de fixation liés aux pattes 3 ont en outre été prévus.

Ces moyens peuvent consister en des crochets formés d'une seule pièce avec les lames (figure 2). Pour favoriser l'adaptation de la prothèse au vaisseau, quelle qu'en soit la courbure, tout ou partie des pattes 3 est réalisé en plusieurs tronçons, par exemple au nombre de trois, tels que figurés en 3a, 3b, 3c sur la figure 4, deux tronçons successifs adjacents étant réunis entre eux par des moyens d'articulation 20 liés aux tronçons correspondants. Les moyens d'articulation pourront notamment consister en des ressorts hélicoïdaux ou en des lames élastiques de flexion, métalliques, pouvant être soudés aux extrémités correspondantes des pattes, en leur permettant de possibles courbures ou des déplacements relatifs transversaux. Eventuellement, les pattes pourraient même être réalisées chacune en une seule pièce, avec des zones intermédiaires d'articulation formant ressort. Par exemple dans le cas de pattes en fil ronds, chaque fil pourrait localement, en deux ou trois zones intermédiaires, être enroulé en hélice, le fil retrouvant sa forme sensiblement rectiligne entre ces zones.

Quelle que soit la forme des pattes, on prévoit par ailleurs dans l'invention d'utiliser, pour leur mouvement relatif radial, des moyens-ressort d'expansion et de rétrécissement radiaux autres que les lamelles 15.

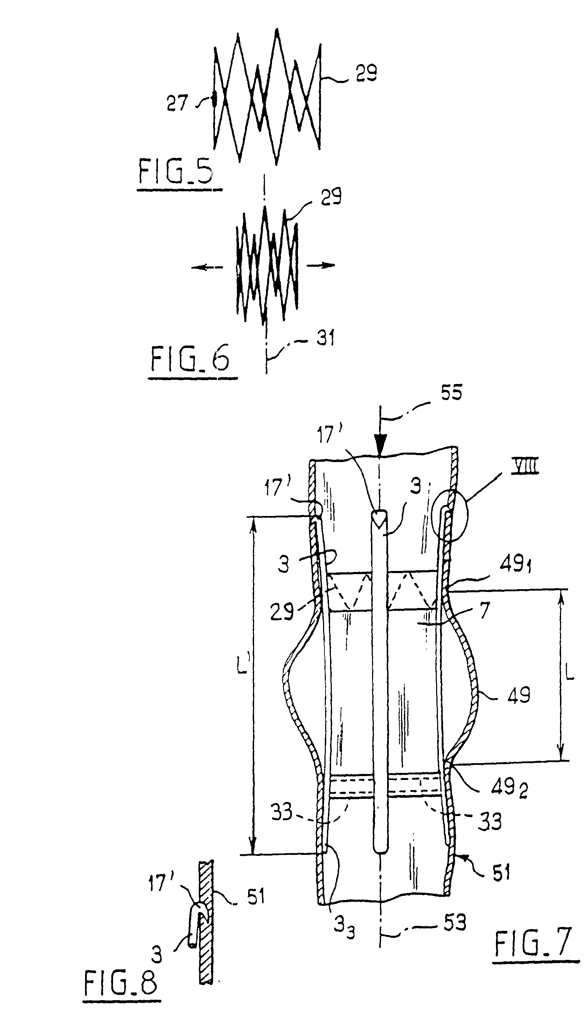

Il est ainsi conseillé de fixer, au moins vers l'une des extrémités de la prothèse, le moyen-ressort 29 illustré sur les figures 5 et 6 en particulier. Il s'agit d'un fin fil relativement rigide (quelques dixièmes de millimètre) en particulier métallique (acier inoxydable par exemple), conformé en zigzags et refermé sur lui-même en 27 pour constituer un cylindre annulaire (figure 5). On notera que ce ressort 29 est, sur cette figure 5, montré dans son état normal non contraint (déployé) tandis qu'il est représenté dans un état contraint sur la figure 6 avec ses tronçons de fil en zigzags plus rapprochés les uns des autres, presque parallèles à l'axe de cylindre 31. Ainsi, la configuration en zigzags sera constituée par un fil présentant une suite de lignes plus ou moins écartées les unes par rapport aux autres angulairement et reliées par des portions d'extrémités courbées.

Intéressons-nous maintenant aux figures 7 et 8 pour décrire l'utilisation d'une forme de réalisation de la prothèse de l'invention qui pourrait être considérée comme la plus appropriée dans le cadre d'une intervention sur un anévrisme important, se manifestant ici par une grosse déformation avec gonflement, en 49, de la paroi du vaisseau 51.

En pratique, le diamètre d'ouverture du manchon sera choisi en fonction de celui du vaisseau. les interventions pouvant en particulier s'effectuer sur une aorte humaine abdominale, les diamètres de la prothèse déployée pourront être compris entre environ 14 et 22 millimètres, la longueur des pattes 3 pouvant être de l'ordre de 8 à 10 centimètres.

Si l'implantation de la prothèse s'effectue par voie percutanée, on utilisera de préférence un cathéter tubulaire d'introduction d'un diamètre par exemple d'environ 6 à 8 millimètres renfermant la prothèse dans son état "replié", avec ici ses lames 3 rapprochées les unes des autres et ses moyens-ressort dans leur état contraint ou resserré.

Si l'on suppose que la flèche 55 de la figure 7 indique le sens de circulation du flux sanguin, le cathéter introducteur (renfermant toujours la prothèse) sera avancé de préférence au moins légèrement au-delà de l'extrémité habituellement dénommée proximale 49₁ de la zone 49.

A l'aide d'une tige-poussoir intérieure glissée à l'intérieur du cathéter introducteur, depuis l'extérieur du corps, on pourra alors pousser la prothèse de telle manière que ses pattes commencent à sortir de l'introducteur en s'ouvrant naturellement, à la manière d'une corolle, le ressort 29 étant disposé avec son axe de cylindre parallèle ou confondu avec celui 53 de la prothèse.

Un retrait progressif du cathéter introducteur dans le sens de la flèche 55 permettra ensuite le déploiement d'ensemble de la prothèse qui, grâce aux crochets d'extrémité 17', pourra s'arrimer à la paroi du vaisseau (figure 8).

Il est conseillé de prévoir une longueur L' de manchon au moins légèrement supérieure à la longueur L de la zone fragilisée 49. Les pattes de stabilisation 3 s'étendent sur toute la longueur du manchon, évitant que la pression du sang le fasse gonfler exagérément.

En ce qui concerne leur nombre, les pattes de stabilisation 3 pourront être par exemple quatre ou six, ce nombre étant adapté au vaisseau sur lequel il faut intervenir.

Par ailleurs, suivant les dimensions axiales de la prothèse, et en particulier si l'on prévoit d'utiliser en tant que moyen d'expansion des pattes 3, uniquement le ressort en zigzags, il pourra s'avérer nécessaire d'intégrer ce ressort à un ou plusieurs endroits de long de la gaine, entre ses extrémités.

## Revendications

1. Dispositif en matériau biocompatible adapté pour être introduit dans un vaisseau, pour le traitement d'un anévrisme, présentant un axe (5) et comprenant
- un manchon souple, allongé (7) présentant un passage intérieur (9) pour canaliser à travers lui le sang,
- des moyens-ressorts (29) liés à des moyens-supports (3) du manchon, pour déployer radialement le manchon et ces moyens-supports, depuis un premier état radialement resserré vers un second état radialement écarté, les moyens-ressorts étant disposés à au moins deux étages axialement éloignés entre lesquels s'étendent les moyens-supports en plusieurs endroits régulièrement répartis autour de l'axe (5), certains au moins desdits moyens-ressorts (29) présentant une configuration fermée en zigzag définissant un cylindre annulaire axial,
**caractérisé en ce que** plusieurs parmi les moyens-supports (3) présentent longitudinalement plusieurs tronçons, deux tronçons (3a, 3b, 3c) successifs adjacents étant réunis entre eux par des moyens d'articulation (20), ou par des zones intermédiaires d'articulation, indépendant(e)s du manchon souple (7), pour articuler le dispositif.

## Patentansprüche

1. Vorrichtung aus einem bioverträglichen Material für das Einführen in ein Gefäß zur Behandlung eines Aneurismas, die eine Achse (5) aufweist und die besteht aus:
- einer weichen Hülse (7) mit einem inneren Durchlass (9) um das Blut hindurch zu führen,
- Federmitteln (29) die mit Haltemitteln (3) der Hülse verbunden sind, um Hülse und die Haltemittel radial von einem ersten, radial zusammengezogenen Zustand zu einem zweiten, geöffneten Zustand aufzuspreizen, wobei die Federmittel auf wenigstens zwei axial auseinanderliegende Niveaus verteilt sind, zwischen denen sich die Haltemittel an mehreren, regelmäßig um die Achse (5) angeordneten Stellen erstrecken, wobei wenigstens einige der Federmittel (29) eine geschlossene Zickzackform aufweisen und einen axialen ringförmigen Zylinder definieren,
**dadurch gekennzeichnet**, dass mehrere der Haltemittel (3) in Längsrichtung mehrere Teilstücke aufweisen, wobei zwei aufeinander folgende Teilstücke (3a, 3b, 3c) durch gelenkige Mittel (20) miteinander verbunden sind, oder durch gelenkige Zwischenzonen, die jeweils unabhängig von der weichen Hülse (7) sind, um die Vorrichtung gelenkig zu machen.

## Claims

1. A device made of a biocompatible material adapted to be introduced in a blood vessel, for treating an aneurysm, the device having an axis (5) and comprising
- a flexible, elongated sleeve (7) having a inner passage (9) for canalizing blood therethrough,
- spring means (29) connected to sleeve support means (3), for radially expanding the sleeve and said support means, from a first radially restricted state to a second radially expanded state, the spring means being arranged,at least at two axially spaced stages between which the support means are disposed, at a plurality of locations, said plurality of locations being regularly located around the axis (5), at least some of said spring means (29) having a closed, zigzag-shape out-line defining an annular, axial cylinder,
- **characterized in that** a plurality of said support means (3) have a plurality of longitudinal sections, two successive, adjacent sections (3a, 3b, 3c) being connected one to the other by articulating means (20), or through intermediate articulating zones, which are independent from the flexible sleeve (7), for articulating the device.
